# EUROPEAN PATENT APPLICATION

(11) **EP 1 275 386 A1**
(43) Date of publication of application: **15.01.2003**
(21) Application number: 01919798.7
(22) Date of filing: 05.04.2001
(51) Int. Cl.: A61K 31/166, A61K 31/277, A61K 31/437, A61K 9/16, A61K 9/20, A61K 47/38, A61K 47/32, A61K 47/34, C07C 237/32, C07C 255/58

(54) **SOLID DISPERSION WITH IMPROVED ABSORBABILITY**

(30) Priority: 19.04.2000 JP 2000118033
(71) Applicant: FUJISAWA PHARMACEUTICAL CO., LTD., Osaka-shi Osaka 541-8514 (JP)
(72) Inventor: HIROSE, Takeo, c/o FUJISAWA PHAR. CO., LTD., Osaka-shi, Osaka 541-8514 (JP); KINOSHITA, Yoshiko, c/o FUJISAWA PHAR. CO., LTD., Osaka-shi, Osaka 541-8514 (JP); SHIMOJO, Fumio, c/o FUJISAWA PHAR. CO., LTD., Osaka-shi, Osaka 541-8514 (JP); OIKE, Atsuo, c/o FUJISAWA PHAR. CO., LTD., Osaka-shi, Osaka 541-8514 (JP)
(74) Representative: Gille Hrabal Struck Neidlein Prop Roos
(86) International application number: JP0102965
(87) International publication number: WO01078716

(57) **Abstract**

A solid dispersion which comprises a polymeric carrier and either a compound (I) represented by the formula (I) or a salt thereof. Thus, the compound (I) and its salt, which are sparingly water-soluble, can have improved oral absorbability.

## Description

### Technical Field

The present invention relates to a solid dispersion formed of a compound represented by the formula (I) described later (hereinafter referred to as a "compound (I)") or a salt thereof and a polymeric carrier, and to a pharmaceutical preparation containing the solid dispersion. The compound (I) of the present invention is useful, for example, as a cGMP-phosphodiesterase inhibitor for treatment of angina pectoris, hypertension, erectile dysfunction and the like.

### Background Art

The compound represented by the formula (I) described later and a salt thereof include those disclosed in International Patent Application Publications WO99/54284 and WO01/05770, and can be manufactured in the manner as described in these publications.

However, the compound (I) is poor in solubility in water, so that it is hardly absorbable when it is orally administered in the form of crystals or micropowders. Improvement of its absorbability has been demanded.

On the other hand, Published Japanese Translation of PCT International Publication for Patent Application No. Hei 9(1997)-501150 discloses a capsule preparation containing a slightly water-soluble compound or a salt thereof, a carrier solution and/or a surfactant and/or a cellulose derivative. According to this preparation, the slightly water-soluble compound becomes microcrystals when it comes out of the capsule since its crystal growth is inhibited. As a result, the preparation shows great bioavailability when administered orally.

However, in the preparation obtained by this method, since an active ingredient is dissolved in a carrier solution in the capsule, the amount of the active ingredient containable in a unit dose preparation is limited.

It is known that, if crystals of a slightly soluble compound are melted with heating to its melting point or higher and then are cooled to the melting point or lower, the slightly soluble compound becomes amorphous and its solubility sometimes improves.

For this reason, the inventors of the present invention sought to obtain an amorphous form of the compound (I) or a salt thereof by melting its crystals alone. The obtained amorphous form of the compound (I) or its salt exhibited solubility in water about 20 times higher than the compound (I) or its salt as a starting material. However, this high solubility was maintained only for a short time, and further study was required.

### Disclosure of Invention

The inventors of the present invention have found that a solid dispersion
obtained by melting a compound (I) or a salt thereof in the coexistence of a polymeric carrier and solidifying with cooling the resulting melt mixture or
obtained by evaporating an organic solvent from a mixture of the compound (I) or a salt thereof, a polymeric carrier and the organic solvent
unexpectedly presents remarkably improved and sustained water-solubility as well as improved and sustained oral absorbability.

### Best Mode for Carrying Out the Invention

Accordingly, the present invention provides a solid dispersion comprising:
a polymeric carrier; and
a compound represented by the formula (I): 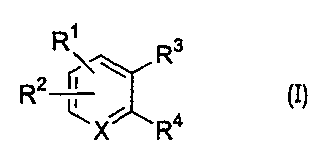 wherein X is CH and other symbols have the following definition 1:
   R¹ is a hydrogen atom or a halogen atom;
   R² is an electron withdrawing group;
   R³ is a group represented by the formula:

      -CONH-A-R¹³

      wherein A is a lower alkylene group; and
      R¹³ is a hydrogen atom; a hydroxy group; a lower alkoxy group; a cycloalkyl group; a substituted or unsubstituted aryl group; or an unsaturated heterocyclic group optionally substituted by lower alkyl; and
   R⁴ is a group represented by the formula:

      -NH-R¹⁴

      wherein R¹⁴ is a lower alkoxy group;
      a substituted or unsubstituted, saturated or unsaturated heterocyclic group;
      an amino group optionally substituted by lower alkyl or halo(lower)alkyl;
      a group represented by the formula: -CH₂-R¹⁵
         wherein R¹⁵ is a cycloalkyl group or an unsaturated heterocyclic group; or
      a group represented by the formula : -CR¹⁶R¹⁷R¹⁸
         wherein R¹⁶ and R¹⁷ are each independently
         a carboxy group;
         a protected carboxy group;
         a carbamoyl group optionally substituted with lower alkyl; or
         a lower alkyl group optionally substituted with one or more substituents selected from the group consisting of halogen; hydroxy; cyano; azido; lower alkoxy; lower alkylthio; protected carboxy; lower alkanesulfonyl; acyloxy; lower alkanesulfonyloxy; aryl; aryloxy which may be substituted by cyano; unsaturated heterocyclic which may be substituted by lower alkyl; guanidino which may be substituted by lower alkyl, cyano and/or halogen; isothioureido which may be substituted by lower alkyl and/or cyano; and amino which may be substituted by acyl, protected carboxy, lower alkanesulfonyl, lower alkanesulfonyloxy or aryloxycarbonyl; or
      R¹⁶ and R¹⁷ together with the carbon atom to which R¹⁶ and R¹⁷ are attached may form a substituted or unsubstituted, saturated carbocyclic group, or an unsaturated carbocyclic group optionally substituted by hydroxy; and
      R¹⁸ is a hydrogen atom; a lower alkoxy group; or a lower alkyl group optionally substituted by hydroxy or lower alkoxy;
or the following definition 2:
R¹ is a hydrogen atom;
R² is a halogen atom; a cyano group; a nitro group; a carbamoyl group; a lower alkylcarbamoyl group which may be substituted by a heterocyclic group; a carboxy group; a protected carboxy group; a lower alkyl group; a halo(lower)alkyl group; a lower alkoxy group; an acyl group; or a lower alkanesulfonyl group; and
R³ and R⁴ together form a group represented by the following formula: 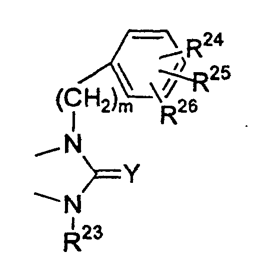
wherein
Y is an oxygen atom or a sulfur atom;
R²³ is a lower alkyl group; a cycloalkyl group; or
   a heterocyclic group,
   among which the lower alkyl group may have one to three substituents selected from the group consisting of hydroxy, protected hydroxy, acyl, lower-alkoxy-substituted aralkyloxy, amino, lower alkylamino, acylamino, lower alkoxycarbonylamino, lower alkanesulfonylamino, ureido, lower alkylureido, sulfamoylamino, protected carboxy, carboxy, lower alkanesulfonyl, lower alkylenedioxy, carbamoyl, lower alkyl carbamoyl and sulfamoyl; and
   the cycloalkyl group and the heterocyclic group may have one to three substituents selected from the group consisting of hydroxy, protected hydroxy, acyl, lower-alkoxy-substituted aralkyloxy, amino, acylamino, lower alkoxycarbonylamino, lower alkanesulfonylamino, ureido, lower alkylureido, sulfamoylamino, protected carboxy, lower alkanesulfonyl, lower alkyl, hydroxy(lower)alkyl, protected hydroxy(lower)alkyl, lower alkylenedioxy, carbamoyl and sulfamoyl;
R²⁴, R²⁵ and R²⁶ are, the same or different,
   a hydrogen atom, a halogen atom, a lower alkanoyl group, a carboxy group, a protected carboxy group, a carbamoyl group, a nitro group, a cyano group, a lower alkyl group, a hydroxy-substituted lower alkyl group, a lower alkoxy group or a lower-alkoxy-substituted aralkyl group; or two of R²⁴, R²⁵ and R²⁶ may combine together to form a lower alkylenedioxy group; and
m is an integer of 1 or 2,
alternatively X is a nitrogen atom and R¹, R², R³ and R⁴ have the same meanings as defined in the above definition 2, or a salt thereof.

The present invention also provides a process for producing a solid dispersion of a compound (I) or a salt thereof comprising evaporating an organic solvent from a mixture of the compound (I) or the salt thereof, a polymeric carrier and an organic solvent, thereby obtaining the solid dispersion of the compound (I) or the salt thereof.

The present invention further provides a process for producing a solid dispersion of a compound (I) or a salt thereof comprising melting the compound (I) or the salt thereof in the coexistence of a polymeric carrier and solidifying with cooling the resulting melt mixture, thereby obtaining the solid dispersion of the compound (I) or the salt thereof.

Regarding the term "solid dispersion" in the present invention, it is considered from an improved bioavailability and a behavior in water of the solid dispersion that the solid dispersion does not form a mere mixture with the polymeric carrier, but forms a specific physical state, that is, a state in which the compound (I) exists in a stable amorphous form on the polymeric carrier.

The compound (I) of the present invention includes the following groups, that is, an anthranilic acid derivative (Ia) represented by the following formula (Ia): 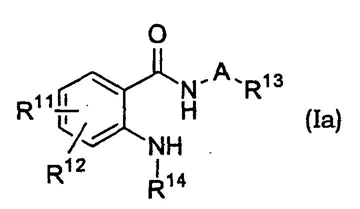 wherein R¹¹ and R¹² have the same meanings as R¹ and R² in the above-mentioned definition 1, and A, R¹³ and R¹⁴ have the same meanings as those in the above-mentioned definition 1,
and a condensed imidazole derivative (Ib) represented by the formula (Ib): 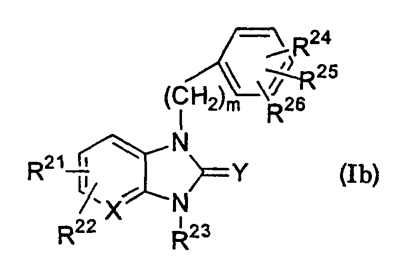 wherein X and CH is nitrogen atom, R²¹ and R²² have the same meanings as R¹ and R² in the above-mentioned definition 2, and Y, R²³, R²⁴, R²⁵, R²⁶ and m have the same meanings as Y, R²³, R²⁴, R²⁵, R²⁶ and m in the above-mentioned definition 2 [this (1b) is further classified into a benzimidazole derivative (Ib-1) (X is CH) and an imidazopyridine derivative (Ib-2) (X is nitrogen atom)].

With respect to details of the anthranilic acid derivative (Ia), its manufacturing method and its pharmacological activity, reference can be made to International Patent Application Publication WO99/54284.

Therefore, the disclosure of International Patent Application Publication WO99/54284 should be construed to be a part of the present specification.

Preferable anthranilic acid derivatives (Ia) are compounds represented by the formula (Ia) wherein R¹¹ is a hydrogen atom; R¹² is a nitro group, a cyano group or a halo(lower)alkyl group; R¹³ is a phenyl group substituted by cyano, lower alkoxy and/or halogen; R¹⁴ is a group represented by -CR¹⁶R¹⁷R¹⁸ wherein R¹⁶ and R¹⁷ are each independently a lower alkyl group optionally substituted by hydroxy, or R¹⁶ and R¹⁷ together with the carbon atom to which R¹⁶ and R¹⁷ are attached form a saturated carbocyclic group substituted by hydroxy, lower alkoxy or acylamino and R¹⁸ is a hydrogen atom; and A is a lower alkylene group.

Among such compounds, particularly preferable ones are
(R)-N-(3,4-dimethoxybenzyl)-2-(2-hydroxy-1-methylethylamino)-5-nit robenzamide,
N-(4-chloro-3-methoxybenzyl)-2-[2-hydroxy-1-(hydroxymethyl)-ethylamino]-5-nitrobenzamide,
N-(3,4-dimethoxybenzyl)-2-[(trans-4-formamidocyclohexyl)-amino]-5-nitrobenzamide dihydrate,
(S)-5-cyano-N-(3,4-dimethoxybenzyl)-2-(2-hydroxy-1-methyl-ethylamino)benzamide and
N-(3-chloro-4-methoxybenzyl)-2-[2-hydroxy-1-(hydroxymethyl)-ethylamino]-5-(trifluoromethyl)benzamide.

With respect to details of the benzimidazole derivative (Ib-1) and the imidazopyridine derivative (Ib-2) in the present invention, their manufacturing method and their pharmacological activity, reference can be made to International Patent Application Publication WO01/05770.

Therefore, the disclosure of International Patent Application Publication WO01/05770 should be construed to be a part of the present specification.

Preferable benzimidazole derivatives (Ib-1) and imidazopyridine derivatives (Ib-2) are compounds represented by formula (Ib) wherein X is a nitrogen atom, Y is an oxygen atom, R²¹ is a hydrogen atom, R²² is a cyano group, R²³ is a cyclohexyl group substituted by hydroxy or lower alkanoyloxy, R²⁴ is a hydrogen atom, R²⁵ is a halogen atom and R²⁶ is a lower alkoxy group.

Among such compounds, are particularly preferable ones are 1-(3-chloro-4-methoxybenzyl)-6-cyano-3-(trans-4-hydroxycyclohexyl)-2,3-dihydro-1H-imidazo[4,5-b]pyridin-2-one, 1-(3-bromo-4-methoxybenzyl)-6-cyano-3-(trans-4-hydroxycyclohexyl)-2,3-dihydro-1H-imidazo[4,5-b]pyridin-2-one and 1-(3-chloro-4-methoxybenzyl)-6-cyano-3-(cis-4-hydroxycyclohexyl)-2,3-dihydro-1H-imidazo[4,5-b]pyridin-2-one.

The compound (I) may be in the form of a salt. Examples of the salts include pharmaceutically acceptable salts, e.g., alkali metal salts (sodium salts, potassium salts and the like), alkali earth metal salts (calcium salts, magnesium salts and the like), ammonium salts, salts with an organic base (trimethylamine, triethylamine, pyridine, picoline, dicyclohexylamine, dibenzylethylenediamine or the like), salts with an organic acid (acetic acid, benzoic acid, succinic acid, fumaric acid, maleic acid, lactic acid, citric acid, tartaric acid, gluconic acid, methanesulfonic acid, benzenesulfonic acid, formic acid, p-toluenesulfonic acid, trifluoroacetic acid or the like), salts with an inorganic acid (hydrochloric acid, hydrobromic acid, sulfuric acid, phosphoric acid or the like) and salts with an amino acid (arginine, aspartic acid, glutamic acid or the like).

The compound (I) and its salt used as a starting material in the present invention may be in the form of solvate such as hydrate, alcoholate and the like. The compound (I) and its salt in such forms may be crystalline, finely crystal-powdered or amorphous.

The polymeric carrier used in the present invention may be any pharmaceutically acceptable one. Examples thereof include cellulose derivatives such as hydroxypropyl cellulose, hydroxypropylmethyl cellulose, methyl cellulose, carboxymethyl cellulose and the like, ethylene glycol polymers such as polyethylene glycol having an average molecular weight of 4,000 or more (e.g., polyethylene glycol 6000), polyvinyl pyrrolidone and the like. Among these, hydroxypropylmethyl cellulose and polyvinyl pyrrolidone are preferred, and hydroxypropylmethyl cellulose is particularly preferred.

The above-described polymeric carriers may be used solely or in combination of two or more thereof.

The content of the compound (I) or its salt in the solid dispersion of the present invention is not particularly limited and may be selected as appropriate depending upon its elution pattern and its action duration time.

The content of the compound (I) or its salt and the polymeric carrier in the solid dispersion depends upon the type of the polymeric carrier, but usually their proportion may be 0.5 to 10 parts by weight of the polymeric carrier, preferably 1 to 5 parts by weight, more preferably 2 to 3 parts by weight, with respect to 1 part by weight of the compound (I) or its salt.

The solid dispersion of the present invention can be produced by a process of evaporating an organic solvent from a mixture of the compound (I) or its salt, the polymeric carrier and the organic solvent.

The organic solvent preferably has such a low boiling point that it easily evaporates and causes little adverse effect on living bodies. For example, may be mentioned lower alkanol such as propanol, ethanol and methanol, acetone, acetonitrile, chloroform, dichloromethane, hexane, toluene, ethyl acetate, methyl ethyl ketone and the like. Among these solvents, ethanol and acetone are preferred. These solvents may be used singly or as a combination of two or more thereof.

For producing the solid dispersion by the above-mentioned process, the compound (I) or its salt is required to be dissolved in the organic solvent. For this purpose, the organic solvent is preferably used in a sufficient amount for dissolving the compound (I) or its salt. Subsequently the polymeric carrier is dissolved or dispersed in the thus obtained solution of the compound (I) to prepare a mixture. The solvent can be evaporated from the mixture in atmospheric pressure to a vacuum in a cooled to heated condition. Ordinarily, it is preferably evaporated under reduced pressure in a heated condition.

Alternatively, the solid dispersion of the present invention can be produced by a process of melting the compound (I) or its salt in the co-existence of the polymeric carrier and solidifying the obtained melt mixture with cooling.

The compound (I) or its salt is melted in the co-existence of the polymeric carrier by heating at a temperature higher than the melting point of the compound (I) or its salt and lower than the decomposition point of the compound (I) or its salt and the polymeric carrier, e.g., at about 140 to 180 °C, for about 30 minutes to 2 hours. For producing the solid dispersion of the present invention, the compound (I) or its salt is required to be melted completely, but the polymeric carrier need not be melted.

The melt mixture can be solidified with cooling to around room temperature by allowing it to cool down or by placing it in a cold place.

In the above-mentioned processes, in the process of evaporating the organic solvent to obtain the solid dispersion, the mixture before the evaporation of the organic solvent may contain generally used additives such as an excipient, a binder, a disintegrator, a surfactant and the like. Also in the process of solidifying with cooling the melt mixture to obtain the solid dispersion, the mixture before the solidification with cooling may contain the above-mentioned additives.

The thus obtained solid dispersion may preferably be pulverized into fine powder if necessary.

The solid dispersion of the present invention may be formulated into pharmaceutical preparations for oral administration such as powder, tablets, capsules, granules, subtilized granules and the like by conventional methods together with pharmaceutically acceptable conventional additives such as an excipient, a binder, a disintegrator, a surfactant and the like. The polymeric carrier used for producing the solid dispersion of the present invention may be used as an additive.

As excipients, may be mentioned lactose, sucrose, glucose, sodium bicarbonate, saccharose, mannitol, starch, crystal cellulose, calcium sulfate, calcium phosphate, ethyl cellulose, methacrylic acid copolymer and the like.

As binders, may be mentioned gum Arabic, polyvinyl pyrrolidone, hydroxypropyl cellulose, hydroxypropylmethyl cellulose, carboxymethyl cellulose, gelatin, glucose, sucrose, gum tragacanth, sodium alginate and the like.

As disintegrators, may be mentioned starch, crystal cellulose, carboxymethyl cellulose, carboxymethyl starch sodium, carboxymethyl cellulose sodium, croscarmellose sodium, crospovidone, low-substituted hydroxypropylcellulose and the like.

As surfactants, may be mentioned polyoxyethylene alkylether, polyoxyethylene sorbitan fatty acid ester, polyoxyethylene glyceryl monofatty acid ester, polyoxyethylenepropyleneglycol monofatty acid ester, polyoxyethylenesorbitol fatty acid ester, polyoxyethylene derivatives of natural oils, fats and waxes, polyethyleneglycol fatty acid ester, sorbitan fatty acid ester, sucrose fatty acid ester, surfactants of polyoxyethylene-polyoxypropylene copolymer and polyoxyethylene-polyoxypropylene block copolymer, alkylsulfate, phospholipid, bile salt, fatty acid, monoalcohol fatty acid ester, ethyleneglycol fatty acid ester, polyalcohol fatty acid ester and the like.

The compound (I) of the present invention is useful as a cGMP-phosphodiesterase inhibitor, and more particularly, useful for the treatment and/or prevention of various diseases, such as angina pectoris, hypertension, pulmonary hypertension, congestive heart failure, glomerular diseases (e.g., diabetic glomerulosclerosis), renal tubulo-intertitial diseases (e.g., nephropathy induced by tacrolimus, cyclosporin or the like), renal failure, atherosclerosis, angiostenosis (e.g., post-percutaneous transluminal coronary angioplasty), peripheral vascular disease, stroke, chronic reversible obstructive lung diseases (e.g., bronchitis or asthma (chronic asthma, allergic asthma)), allergic rhinitis, urticaria, glaucoma, diseases characterized by disorders of gut motility (e.g., irritable bowel syndrome), erectile dysfunction (e.g., organic erectile dysfunction or psychic erectile dysfunction), female sexual dysfunction, impotence, or diabetic complications (e.g., diabetic gangrene, diabetic arthropathy, diabetic glomerulosclerosis, diabetic dermopathy, diabetic neuropathy, diabetic cataract or diabetic retinopathy).

While the dosage of the compound (I) varies depending upon the age and condition of each individual patient to be treated, in case of the systemic administration, a daily dose of about 0.01-1000 mg, preferably 0.2-500 mg and more preferably 0.5-100 mg is generally given, and a single dose of about 0.01-0.5 mg, 1 mg, 5 mg, 10 mg, 50 mg, 100 mg, 250 mg and 500 mg is generally administered. Daily doses for chronic administration will be in the range of about 0.3 mg to 1,000 mg.

### EXAMPLES

The present invention is now described with reference to the following examples, but the present invention is not limited thereto.

### Production Example 1

In a solution of 2-[(trans-4-aminocyclohexyl)amino]-N-(3,4-dimethoxybenzyl)-5-nitrobenzamide (10 g) in dimethylformamide (60 mL), ethyl formate (200 mL) was added. The resulting mixture was refluxed with heating for 8 hours. The mixture was distributed in ethyl acetate and water. The separated organic phase was washed with water and brine, dried over magnesium sulfate and evaporated under vacuum. The residue was purified by silica gel column chromatography eluting with a mixture of chloroform and methanol (20 : 1). The obtained product was recrystallized from ethanol to obtain a yellow crystal of N-(3,4-dimethoxybenzyl)-2-(trans-4-formamidocyclohexyl)amino]-5-nitrobenzamide dihydrate (7.21g). This was a dihydrate crystal.
NMR (DMSO-d₆, δ ) : 1.25-1.55 (4H, br), 1.79-1.92 (2H, br), 1.95-2.10 (2H, br), 3.47-3.59 (1H, br), 3.60-3.72 (1H, br), 3.73 (3H,s), 3.74 (3H, s), 4.37 (2H, d, J = 7Hz), 6.85-6.96 (4H, m), 7.95 (1H, s), 8.04 (1H, br), 8.08 (1H, dd, J = 4,8Hz), 8.60 (1H, d, J = 4Hz), 9.01 (1H, d, J = 8Hz), 9.32 (1H, br)
Mass m/z : 455(M+).

### Example 1

(R)-N-(3,4-Dimethoxybenzyl)-2-(2-hydroxy-1-methylethyl amino)-5-nitrobenzamide (1.0 g) and hydroxypropyl cellulose (3.0 g) were mixed. The resulting mixture was heated in an oven at 150 °C for 30 minutes to melt the active component. The resulting mixture was allowed to cool to room temperature, and the obtained solid was pulverized in a mortar and sieved through a No. 30 mesh to obtain a solid dispersion.

### Example 2

N-(3,4-Dimethoxybenzyl)-2-[(trans-4-formamidocyclohexyl) amino]-5-nitrobenzamide dihydrate (1.0 g) and hydroxypropylmethyl cellulose (3.0 g) were mixed. The resulting mixture was heated in an oven at 165 °C for 30 minutes to melt the active component. The mixture was allowed to cool to room temperature, and the obtained solid was pulverized in a mortar and sieved through a No. 30 mesh to obtain a solid dispersion.

### Example 3

N-(3,4-Dimethoxybenzyl)-2-[(trans-4-formamidocyclohexyl)-amino]-5-nitrobenzamide dihydrate (1.0 g) and polyvinyl pyrrolidone (3.0 g) were mixed. The resulting mixture was heated in an oven at 175 °C for 30 minutes to melt the active component. The mixture was allowed to cool to room temperature, and the obtained solid was pulverized in a mortar and sieved through a No. 30 mesh to obtain a solid dispersion.

### Example 4

N-(4-Chloro-3-methoxybenzyl)-2-[2-hydroxy-1-(hydroxy methyl)ethylamino]-5-nitrobenzamide (1.0 g) and polyethylene glycol 6000 (3.0 g) were mixed. The resulting mixture was heated in an oven at 165 °C for 30 minutes to melt the active component. The mixture was allowed to cool to room temperature, and the obtained solid was pulverized in a mortar and sieved through a No. 30 mesh to obtain a solid dispersion.

### Example 5

N-(3,4-Dimethoxybenzyl)-2-[(trans-4-formamidocyclohexyl) amino]-5-nitrobenzamide dihydrate (1.0 g) was dissolved in a mixture liquid (100 mL) of ethanol and acetone (1 : 2). In the resulting solution, hydroxypropyl cellulose (3.0 g) was dissolved, and then the solvent was evaporated out by use of a rotary evaporator. The residue was dried under vacuum overnight, and the resulting solid was pulverized in a mortar and sieved through a No. 30 mesh to obtain a solid dispersion.

### Example 6

(S)-5-Cyano-N-(3,4-dimethoxybenzyl)-2-(2-hydroxy-1-methy lethylamino)benzamide (1.0 g) was dissolved in a mixture liquid (100 mL) of ethanol and acetone (1 : 2). In the resulting solution, hydroxypropylmethyl cellulose (3.0 g) was dispersed, and then the solvent was evaporated out by use of a rotary evaporator. The residue was dried under vacuum overnight, and the resulting solid was pulverized in a mortar and sieved through a No. 30 mesh to obtain a solid dispersion.

### Example 7

N-(3-Chloro-4-methoxybenzyl)-2-[2-hydroxy-1-(hydroxy methyl)ethylamino)-5-(trifluoromethyl)benzamide (1.0 g) was dissolved in a mixture liquid (100 mL) of ethanol and acetone (1 : 2). In the resulting solution, polyvinyl pyrrolidone (3.0 g) was dissolved, and then the solvent was evaporated out by use of a rotary evaporator. The residue was dried under vacuum overnight, and the resulting solid was pulverized in a mortar and sieved through a No. 30 mesh to obtain a solid dispersion.

### Example 8

(R)-N-(3,4-Dimethoxybenzyl)-2-(2-hydroxy-1-methylethyl amino)-5-nitrobenzamide(1.0 g) was dissolved in a mixture liquid (100 mL) of ethanol and acetone (1 : 2). In the resulting solution, polyethylene glycol 6000 (3.0 g) was dissolved, and then the solvent was evaporated out by use of a rotary evaporator. The residue was dried under vacuum overnight, and the resulting solid was pulverized in a mortar and sieved through a No. 30 mesh to obtain a solid dispersion.

### Example 9

N-(3,4-Dimethoxybenzyl)-2-[(trans-4-formamidocyclohexyl)-amino]-5-nitrobenzamide dihydrate (1.0 g) and hydroxypropylmethyl cellulose (2.0 g) were mixed. The resulting mixture was heated in an oven at 165 °C for 30 minutes to melt the active component. The mixture was allowed to cool to room temperature, and the obtained solid was pulverized in a mortar and sieved through a No. 30 mesh to obtain a solid dispersion.

### Example 10

N-(3,4-Dimethoxybenzyl)-2-[(trans-4-formamidocyclohexyl)-amino]-5-nitrobenzamide dihydrate (1.0 g), hydroxypropylmethyl cellulose (2.0 g), mannitol (1.0 g), crospovidone (1.0 g) and polyoxyl 40 stearate (0.1 g) were mixed. The resulting mixture was heated in an oven at 165 °C for 30 minutes to melt the active component. The mixture was allowed to cool to room temperature, and the obtained solid was pulverized in a mortar and sieved through a No. 30 mesh to obtain a solid dispersion.

### Example 11

1-(3-Chloro-4-methoxybenzyl)-6-cyano-3-(trans-4-hydroxy cyclohexyl)-2,3-dihydro-1H-imidazo[4,5-b]pyridin-2-one (1.0 g) and hydroxypropylmethyl cellulose (3.0 g) were mixed. The resulting mixture was heated in an oven at 170 °C for 30 minutes to melt the active component. The mixture was allowed to cool to room temperature, and the obtained solid was pulverized in a mortar and sieved through a No. 30 mesh to obtain a solid dispersion.

### Example 12

1-(3-Bromo-4-methoxybenzyl)-6-cyano-3-(trans-4-hydroxy cyclohexyl)-2,3-dihydro-1H-imidazo[4,5-b]pyridin-2-one (1.0 g) and polyvinyl pyrrolidone (3.0 g) were mixed. The resulting mixture was heated in an oven at 165 °C for 30 minutes to melt the active component. The mixture was allowed to cool to room temperature, and the obtained solid was pulverized in a mortar and sieved through a No. 30 mesh to obtain a solid dispersion.

### Example 13

1-(3-Chloro-4-methoxybenzyl)-6-cyano-3-(cis-4-hydroxy cyclohexyl)-2,3-dihydro- 1H-imidazo[4,5-b]pyridin-2-one was dissolved in a mixture liquid (100 mL) of ethanol and acetone (1 : 2). In the resulting solution, polyethylene glycol 6000 (3.0 g) was dissolved, and then the solvent was evaporated out by use of a rotary evaporator. The residue was dried under vacuum overnight, and the resulting solid was pulverized in a mortar and sieved through a No. 30 mesh to obtain a solid dispersion.

Differential scanning calorimetry revealed that the active ingredients in the solid dispersions obtained in the above Examples 1 to 13 were all amorphous.

### Example 14

To the solid dispersion (1 g) obtained in Example 9, low-substituted hydroxypropylcellulose (2 g), magnesium stearate (0.02 g) and lactose (in a proper amount) were added so that the total amount was 6 g, and were mixed with stirring. Thereafter, the resulting mixture was compressed into tablets so that the amount of the active ingredient was 180 mg per tablet.

### Example 15

To the solid dispersion (1g) obtained in Example 9, magnesium stearate (0.117 g) and lactose (in a proper amount) were added so that the total amount was 11.7 g, and were mixed with stirring. Thereafter, the resulting mixture was packed in No. 1 capsules to obtain capsules each containing 350 mg of the active ingredient.

### Example 16

To the solid dispersion (1g) obtained in Example 9, lactose (15.67 g) was added and mixed with stirring. Thereafter, the resulting mixture was dry-granulated and subjected to size selection to obtain granules.

### Comparative Example 1

N-(3,4-Dimethoxybenzyl)-2-[(trans-4-formamidocyclohexyl) amino]-5-nitrobenzamide dihydrate was heated for 3 minutes at a temperature (about 220 °C) higher than its melting point and then allowed to cool to room temperature. The obtained solid was amorphous.

### Test Example 1 (Test on Solubility of Active Component)

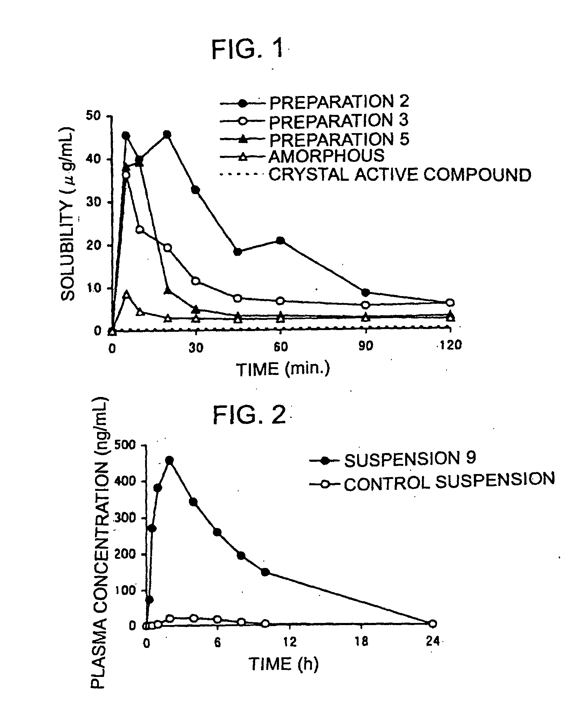

Compared was the solubility of the solid dispersions obtained in Examples 2, 3 and 5 (referred to as "Preparation 2," "Preparation 3" and "Preparation 5," respectively, hereinafter) containing as an active ingredient N-(3,4-dimethoxybenzyl)-2-[(trans-4-formamidocyclohexyl)amino]-5-nitrobenzamide dihydrate (referred to as "Active Component 1" hereinafter), the amorphous Active Component 1 obtained in Comparative Example 1 and the crystal Active Component 1 obtained in Production Example 1. The test was performed by adding Preparations 2, 3 and 5, the amorphous Active Component 1 and the crystal Active Component 1 each containing 15mg equivalent of Active Component 1 to 7.5 mL of water heated to 37 °C, agitating the resulting mixtures with a stirrer and measuring the concentration of Active Component 1 in the solutions. The results are shown in Fig. 1, which is a graphical representation showing the change with time of the solubility in water of Preparations 2, 3 and 5, the amorphous Active Component 1 and the crystal Active Component 1. Also Table 1 shows the maximum concentration achieved of Active Component 1 of each of the solutions in water.

**Table 1**

| | Maximum Concentration Achieved of Active Component 1 in water (µg/mL) |
|---|---|
| Preparation 2 containing Active Component 1 | 45. 46 |
| Preparation 3 containing Active Component 1 | 36. 22 |
| Preparation 5 containing Active Component 1 | 39.17 |
| Amorphous Active Component 1 | 8. 68 |
| Crystal Active Component 1 | 0. 38 |

As clearly understood from Fig. 1 and Table 1, Preparations 2, 3 and 5 exhibited better solubility than the crystal Active Component 1 and the amorphous Active Component 1. It was observed that Preparation 2 exhibited particularly high solubility and was excellent in maintaining the solubility.

### Test Example 2 (Test on Crystal State of Active Component 1)

Preparations 2 and 3 containing Active Component 1 and the amorphous Active Component 1 after being stored at 70 °C for 7 days were tested on the crystal state of Active Component 1 by differential scanning calorimetry (DSC for short hereinafter). The results are shown in Table 2. It was suggested that the amorphous Active Component 1 not containing the polymeric carrier changed from the amorphous state to a crystal state after being stored at 70 °C for 7 days, while it was confirmed that Preparations 2 and 3 did not change from the amorphous state to the crystal state after being stored at 70 °C for 7 days and were stable in the amorphous state.

**Table 2**

| | | D S C analysis results |
|---|---|---|
| Preparation 2 | Before storage | Endothermic peak caused by melting indicative of existence of crystal was not recognized. |
| | After storage | Endothermic peak due to melting indicative of existence of crystal was not recognized. |
| Preparation 3 | Before storage | Endothermic peak caused by melting indicative of existence of crystal was not recognized. |
| | After storage | Endothermic peak caused by melting indicative of existence of crystal was not recognized. |
| Amorphous A | Before storage | Endothermic peak caused by melting indicative of existence of crystal was not recognized. |
| | After storage | Endothermic peak caused by melting indicative of existence of crystal was recognized. |

### Test Example 3 (Test on Oral Absorption of Active Component 1)

### (1) Test Preparations

Tested were a suspension (referred to as "Suspension 9" hereinafter) of 480 mg of the preparation obtained in Example 9 (referred to as "Preparation 9" hereinafter) containing as an active ingredient Active Component 1 in 50 mL of water and a suspension (referred to as "Control Suspension" hereinafter) of 160 mg of the crystal Active Component 1 in 50 mL of water.

### (2) Test Method

The above-mentioned Suspension 9 and Control Suspension were orally administered once to male beagles (weight 10 to 12 kg, 3 individuals per group) fasted overnight. The dose was adjusted to 3.2 mg/kg in terms of Active Component 1.

### (3) Test Results

Fig. 2 shows change in mean plasma concentration. Fig. 2 is a graph showing the change in the mean plasma concentration when Suspension 9 and Control Suspension were orally administered. Table 3 shows PK parameters. In the table, Cₘₐₓ represents the maximum plasma concentration, Tₘₐₓ represents a time required for achieving the maximum plasma concentration, and AUC₀₋₂₄ₕᵣ represents area under a plasma concentration/time curve from administration to 24 hours after the administration. Bioavailability (BA) is calculated from AUC obtained by an intravenous administration test carried out separately. The numerical values in the table signify an average value±a standard error.

**Table 3**

| | Samples Cₘₐₓ (ng/mL) | Tₘₐₓ (hours) | AUC₀₋₂₄ₕᵣ (ng·hour/mL) | BA (%) |
|---|---|---|---|---|
| Suspension 9 | 457.4±48.3 | 2.0±0.0 | 3876.5±665.6 | 54.3 |
| Control Suspension | 21.7±0.7 | 3.3±0.7 | 155.8±38.6 | 2.2 |

As clearly understood from Table 3, Suspension 9 containing Preparation 9 exhibited a Cₘₐₓ value and a AUC₀₋₂₄ₕᵣ value each about 20 times higher than those of Control Suspension. The BA value of Suspension 9 demonstrates excellent oral absorbability of Preparation 9.

### Test Example 4 (Test on Crystal State and Solubility of Active Component 1)

After the above-mentioned Preparation 9 was stored at 50 °C for 3 months, the crystal state of Active Component 1 in Preparation 9 was tested by DSC. As a result, the endothermic peak indicative of change from the amorphous state to the crystal state was not recognized in Active Component 1 in Preparation 9 even after 3 months' storage, and it was confirmed that Active Component 1 in Preparation 9 was stable in the amorphous state. Also Preparation 9 was tested on its solubility after being stored at 50 °C for 3 months. The solubility test was carried out in accordance with the second method of the elution test described in Japanese Pharmacopeia 13^{th} edition. That is, 50 mg equivalent of Active Component 1 were added to a test liquid of 900 mL water, and the solubility of Active Component 1 was measured with time under a puddle rotation at 50 rpm. The results are shown in Table 4. As clearly understood from Table 4, the maximum concentration achieved of Active Component 1 did not change before and after storage at 50 °C for 3 months.

**Table 4**

| | Maximum Concentration Achieved of Active Component 1 (µg/mL) |
|---|---|
| Before storage | 37. 80 |
| After storage | 37. 17 |

From these results, it was understood that Active Component 1 also exist stably as amorphous in Preparation 9.

### Industrial Applicability

In the solid dispersion of the present invention containing as the active component the compound (I) or a salt thereof, the compound (I) and its salt can be present stably in the amorphous state for a long time, and therefore, it exhibits very excellent oral absorbability over a long time.

## Claims

1. A solid dispersion comprising:
a polymeric carrier; and
a compound (I) represented by the formula (I): 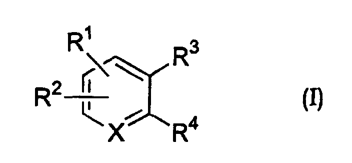 wherein X is CH and other symbols have the following definition 1:
R¹ is a hydrogen atom or a halogen atom;
R² is an electron withdrawing group;
R³ is a group represented by the formula:
-CONH-A-R¹³
wherein A is a lower alkylene group; and
R¹³ is a hydrogen atom; a hydroxy group; a lower alkoxy group; a cycloalkyl group; a substituted or unsubstituted aryl group; or an unsaturated heterocyclic group optionally substituted by lower alkyl; and
R⁴ is a group represented by the formula:
-NH-R¹⁴
wherein R¹⁴ is a lower alkoxy group;
a substituted or unsubstituted, saturated or unsaturated heterocyclic group;
an amino group optionally substituted by lower alkyl or halo (lower) alkyl;
a group represented by the formula: -CH₂-R¹⁵
wherein R¹⁵ is a cycloalkyl group or an unsaturated heterocyclic group; or
a group represented by the formula : -CR¹⁶R¹⁷R¹⁸
wherein R¹⁶ and R¹⁷ are each independently
a carboxy group;
a protected carboxy group;
a carbamoyl group optionally substituted with lower alkyl; or
a lower alkyl group optionally substituted with one or more substituents selected from the group consisting of halogen; hydroxy; cyano; azido; lower alkoxy; lower alkylthio; protected carboxy; lower alkanesulfonyl; acyloxy; lower alkanesulfonyloxy; aryl; aryloxy which may be substituted by cyano; unsaturated heterocyclic which may be substituted by lower alkyl; guanidino which may be substituted by lower alkyl, cyano and/or halogen; isothioureido which may be substituted by lower alkyl and/or cyano; and amino which may be substituted by acyl, protected carboxy, lower alkanesulfonyl, lower alkanesulfonyloxy or aryloxycarbonyl; or
R¹⁶ and R¹⁷ together with the carbon atom to which R¹⁶ and R¹⁷ are attached may form a substituted or unsubstituted, saturated carbocyclic group, or
an unsaturated carbocyclic group optionally substituted by hydroxy; and
R¹⁸ is a hydrogen atom;
a lower alkoxy group; or
a lower alkyl group optionally substituted by hydroxy or lower alkoxy;
or the following definition 2:
R¹ is a hydrogen atom;
R² is a halogen atom; a cyano group; a nitro group; a carbamoyl group; a lower alkylcarbamoyl group which may be substituted by a heterocyclic group; a carboxy group; a protected carboxy group; a lower alkyl group; a halo(lower)alkyl group; a lower alkoxy group; an acyl group; or a lower alkanesulfonyl group; and
R³ and R⁴ together form a group represented by the following formula: 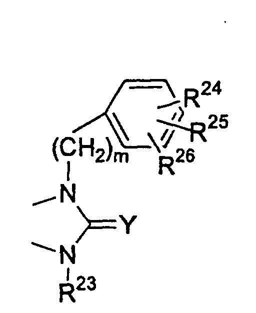
wherein Y is an oxygen atom or a sulfur atom;
R²³ is a lower alkyl group; a cycloalkyl group; or
a heterocyclic group,
among which the lower alkyl group may have one to three substituents selected from the group consisting of hydroxy, protected hydroxy, acyl, lower-alkoxy-substituted aralkyloxy, amino, lower alkylamino, acylamino, lower alkoxycarbonylamino, lower alkanesulfonylamino, ureido, lower alkylureido, sulfamoylamino, protected carboxy, carboxy, lower alkanesulfonyl, lower alkylenedioxy, carbamoyl, lower alkylcarbamoyl and sulfamoyl;
and
the cycloalkyl group and the heterocyclic group may have one to three substituents selected from the group consisting of hydroxy, protected hydroxy, acyl, lower-alkoxy-substituted aralkyloxy, amino, acylamino, lower alkoxycarbonylamino, lower alkanesulfonylamino, ureido, lower alkylureido, sulfamoylamino, protected carboxy, lower alkanesulfonyl, lower alkyl, hydroxy(lower)alkyl, protected hydroxy(lower)alkyl, lower alkylenedioxy, carbamoyl and sulfamoyl;
R²⁴, R²⁵ and R²⁶ are, the same or different,
a hydrogen atom, a halogen atom, a lower alkanoyl group, a carboxy group, a protected carboxy group, a carbamoyl group, a nitro group, a cyano group, a lower alkyl group, a hydroxy-substituted lower alkyl group, a lower alkoxy group or a lower-alkoxy-substituted aralkyl group; or two of R²⁴, R²⁵ and R²⁶ may combine together to form a lower alkylenedioxy group; and
m is an integer of 1 or 2,
alternatively X is a nitrogen atom and R¹, R², R³ and R⁴ have the same meanings as defined in the above definition 2, or a salt of the compound (I).

2. A solid dispersion as recited in claim 1, wherein the compound (I) is an anthranilic acid derivative (Ia) represented by the formula (Ia): 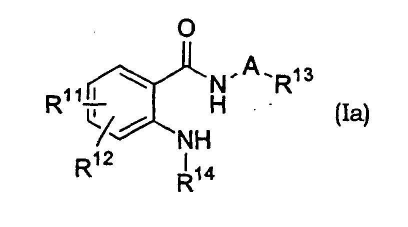 wherein
R¹¹ is a hydrogen atom or a halogen atom;
R¹² is an electron withdrawing group;
R¹³ is a hydrogen atom; a hydroxy group; a lower alkoxy group; a cycloalkyl group; a substituted or unsubstituted aryl group; or an unsaturated heterocyclic group optionally substituted by lower alkyl;
A is a lower alkylene group; and
R¹⁴ is a lower alkoxy group;
a substituted or unsubstituted, saturated or unsaturated heterocyclic group;
an amino group optionally substituted by lower alkyl or halo (lower) alkyl;
a group represented by the formula: -CH₂-R¹⁵ wherein R¹⁵ is a cycloalkyl group or an unsaturated heterocyclic group; or
a group represented by the formula : -CR¹⁶R¹⁷R¹⁸ wherein R¹⁶ and R¹⁷ are each independently
a carboxy group;
a protected carboxy group;
a carbamoyl group optionally substituted by lower alkyl; or
a lower alkyl group optionally substituted by one or more substituents selected from the group consisting of halogen; hydroxy; cyano; azido; lower alkoxy; lower alkylthio; protected carboxy; lower alkanesulfonyl; acyloxy; lower alkanesulfonyloxy; aryl; aryloxy which may be substituted by cyano; an unsaturated heterocyclic group which may be substituted by lower alkyl; guanidino which may be substituted by lower alkyl, cyano and/or halogen; isothioureido which may be substituted by lower alkyl and/or cyano; and amino which may be substituted by acyl, protected carboxy, lower alkanesulfonyl, lower alkanesulfonyloxy or aryloxycarbonyl; or
R¹⁶ and R¹⁷ together with the carbon atom to which R¹⁶ and R¹⁷ are attached may form a substituted or unsubstituted, saturated carbocyclic group, or an unsaturated carbocyclic group optionally substituted by hydroxy, and
R¹⁸ is a hydrogen atom; a lower alkoxy group; or a lower alkyl group optionally substituted by hydroxy or a lower alkoxy.

3. A solid dispersion as recited in claim 2, wherein, in the formula (Ia),
R¹¹ is a hydrogen atom;
R¹² is a nitro group, a cyano group or a halo(lower)alkyl group;
R¹³ is a phenyl group substituted by cyano, lower alkoxy and/or halogen;
R¹⁴ is a group -CR¹⁶R¹⁷R¹⁸ wherein
R¹⁶ and R¹⁷ are each independently a lower alkyl group optionally substituted by hydroxy; or
R¹⁶ and R¹⁷ together with the carbon atom to which R¹⁶ and
R¹⁷ are attached form a saturated carbocyclic group substituted by hydroxy, lower alkoxy or acylamino; and
R¹⁸ is a hydrogen atom; and
A is a lower alkylene group.

4. A solid dispersion as recited in claim 3, wherein the anthranilic acid derivative (Ia) is
(R)-N-(3,4-dimethoxybenzyl)-2-(2-hydroxy-1-methylethylamino)-5-nitrobenzamide;
N-(4-chloro-3-methoxybenzyl)-2-[2-hydroxy-1-(hydroxymethyl)ethylamino]-5-nitrobenzamide;
N-(3,4-dimethoxybenzyl)-2-[(trans-4-formamidocyclohexyl)amino]-5-nitrobenzamide dihydrate,
(S)-5-cyano-N-(3,4-dimethoxybenzyl)-2-(2-hydroxy-1-methylethylamino)benzamide; or
N-(3-chloro-4-methoxybenzyl)-2-[2-hydroxy-1-(hydroxymethyl)ethylamino]-5-(trifluoromethyl)benzamide.

5. A solid dispersion as recited in claim 1, wherein the compound (I) is a condensed imidazole derivative (Ib) represented by the formula (Ib): 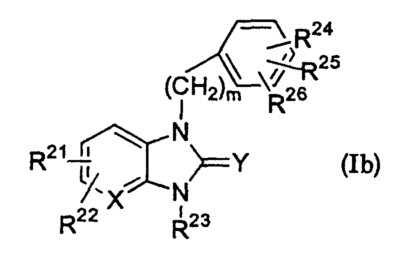 wherein
X is CH or a nitrogen atom;
R²¹ is a hydrogen atom;
R²² is a halogen atom; a cyano group; a nitro group; a carbamoyl group; a lower alkylcarbamoyl group which may be substituted by a heterocyclic group; a carboxy group; a protected carboxy group; a lower alkyl group; a halo(lower)alkyl group; a lower alkoxy group; an acyl group; or a lower alkanesulfonyl group;
Y is an oxygen atom or a sulfur atom;
R²³ is a lower alkyl group, a cycloalkyl group or a heterocyclic group,
among which the lower alkyl group may have one to three substituents selected from the group consisting of hydroxy, protected hydroxy, acyl,
lower-alkoxy-substituted aralkyloxy, amino, lower alkylamino, acylamino, lower alkoxycarbonylamino, lower alkanesulfonylamino, ureido, lower alkylureido, sulfamoylamino, protected carboxy, carboxy, lower alkanesulfonyl, lower alkylenedioxy, carbamoyl, lower alkyl carbamoyl and sulfamoyl; and
the cycloalkyl group and the heterocyclic group may have one to three substituents selected from the group consisting of hydroxy, protected hydroxy, acyl, lower-alkoxy-substituted aralkyloxy, amino, acylamino, lower alkoxycarbonylamino, lower alkanesulfonylamino, ureido, lower alkylureido, sulfamoylamino, protected carboxy, lower alkanesulfonyl, lower alkyl, hydroxy(lower)alkyl, protected hydroxy(lower)alkyl, lower alkylenedioxy, carbamoyl and sulfamoyl;
R²⁴, R²⁵ and R²⁶ are, the same or different, a hydrogen atom, a halogen atom, a lower alkanoyl group, a carboxy group, a protected carboxy group, a carbamoyl group, a nitro group, cyano group, a lower alkyl group, a hydroxy-substituted lower alkyl group, a lower alkoxy group or a lower-alkoxy-substituted aralkyl group; or two of R²⁴, R²⁵ and R²⁶ may combine together to form a lower alkylenedioxy group; and
m is an integer of 1 or 2.

6. A solid dispersion as recited in claim 5, wherein, in the formula (Ib),
X is a nitrogen atom;
R²¹ is a hydrogen atom;
R²² is a cyano group;
Y is an oxygen atom;
R²³ is a cyclohexyl group substituted by hydroxy or lower alkanoyloxy;
R²⁴ is a hydrogen atom;
R²⁵ is a halogen atom; and
R²⁶ is a lower alkoxy group.

7. A solid dispersion as recited in claim 6, wherein the condensed imidazole derivative (Ib) is 1-(3-chloro-4-methoxybenzyl)-6-cyano-3-(trans-4-hydroxycyclohexyl)-2,3-dihydro-1H-imidazo[4,5-b]pyridin-2-one; 1-(3-bromo-4-methoxybenzyl)-6-cyano-3-(trans-4-hydroxycyclohexyl)-2,3-dihydro-1H-imidazo[4,5-b]pyridin-2-one; or 1-(3-chloro-4-methoxybenzyl)-6-cyano-3-(cis-4-hydroxycyclohexyl)-2,3-dihydro-1H-imidazo[4,5-b]pyridin-2-one.

8. A solid dispersion as recited in any one of claims 1 to 7, wherein the polymeric carrier is hydroxypropyl cellulose, hydroxypropylmethyl cellulose, methyl cellulose, carboxymethyl cellulose, polyvinyl pyrrolidone or polyethylene glycol having an average molecular weight of 4,000 or more.

9. A process for producing a solid dispersion of a compound (I) or a salt thereof comprising evaporating an organic solvent from a mixture of the compound (I) or the salt thereof, a polymeric carrier and the organic solvent, thereby obtaining the solid dispersion of the compound (I) or the salt thereof.

10. A process as recited in claim 9, wherein the mixture is obtained by dissolving the compound (I) or the salt thereof in the organic solvent and then dissolving or dispersing the polymeric carrier in the resulting solution.

11. A process as recited in claim 9 or 10, wherein the organic solvent is a lower alkanol, acetone or a mixture thereof.

12. A process of producing a solid dispersion of a compound (I) or a salt thereof comprising melting the compound (I) or the salt thereof in the coexistence of a polymeric carrier and solidifying with cooling the resulting melt mixture, thereby obtaining the solid dispersion of the compound (I) or the salt thereof.

13. A process as recited in claim 12, wherein melting is performed at 140 °C to 180 °C.

14. A process as recited in any one of claims 9 to 13, wherein the compound (I) or the salt thereof used as a starting material is crystalline.

15. A pharmaceutical preparation comprising a solid dispersion of a compound (I) or a salt thereof as recited in any one of claims 1 to 8 and optionally a pharmaceutically acceptable additive.
